# EUROPEAN PATENT SPECIFICATION

(11) **EP 1 409 022 B1**
(45) Date of publication and mention of the grant of the patent: **03.08.2011**
(21) Application number: 01971509.3
(22) Date of filing: 02.10.2001
(51) Int. Cl.: A61P 17/10, A61K 9/12, A61K 47/10

(54) **PHARMACEUTICAL MOUSSE COMPOSITION COMPRISING SALICYLIC ACID**
SALICYLSÄURE ENTHALTENDE PHARMAZEUTISCHE SCHAUMZUSAMMENSETZUNG
COMPOSITION PHARMACEUTIQUE DE MOUSSE COMPRENANT DE L'ACIDE SALICYLIQUE

(30) Priority: 02.10.2000 AU PR048600
(43) Date of publication of application: 21.04.2004
(73) Proprietor: Stiefel Research Australia Pty Ltd, Rowville VIC 3178 (AU)
(72) Inventor: ABRAM, Albert, Wantirna, VIC 3152 (US); HOULDEN, Robert, Kilsyth, VIC 3137 (AU); PIRZAS, Vicky, Blackburn North, VIC 3130 (AU)
(74) Representative: Reeves, Julie Frances
(86) International application number: PCT/AU2001/001237
(87) International publication number: WO 2002/028435

(56) References cited:
- EP-A1- 0 484 530
- EP-B1- 0 331 489
- WO-A-99/53923
- AU-B2- 701 554
- AU-B2- 709 320
- AU-B2- 732 456
- US-A- 5 652 228
- US-A- 5 747 021
- US-A- 5 783 202
- WOODFORD R ET AL: "BIOAVAILABILITY AND ACTIVITY OF TOPICAL CORTICOSTEROIDS FROM A NOVEL DRUG DELIVERY SYSTEM, THE AEROSOL QUICK-BREAK FOAM" January 1977 (1977-01), JOURNAL OF PHARMACEUTICAL SCIENCES, AMERICAN PHARMACEUTICAL ASSOCIATION. WASHINGTON, US, PAGE(S) 99-103 , XP000996069 ISSN: 0022-3549 * page 99, column 2, line 26 - page 100, column 1, line 2 *

## Description

### FIELD OF THE INVENTION

This invention relates to vehicles for the percutaneous delivery of at least one pharmaceutically active agent to the epidermis. In particular this invention relates to pharmaceutical compositions directed to the treatment of skin diseases, more particularly those containing salicylic acid.

### BACKGROUND

Pharmaceutical active agents commonly used to treat acne and other skin diseases include but are not limited to the therapeutic substances salicylic acid, isotretinoin, benzoyl peroxide, resorcinol, non-steroidal anti-inflammatory drugs such as ketoprofen, corticosteroids such as cortisone, antifungals, antibiotics for microbial infections and anti-psoriatics such as etrinate. It is common to also include other substances such as anaesthetics, for example lignocaine where necessary. Treatment of acne is traditionally effected by external, topical application of a pharmaceutical substance. Where this is ineffective, systemic treatments such as by hormone treatment can be utilised but do have undesirable side effects in some patients. Salicylic acid is one well recognized anti-acne active agent which causes a reduction in intercellular cohesion of individual dead skin cells which are the starting point of acne infection. Ideally an anti acne pharmaceutical should maximise penetration of the active agent through the upper layers of the epidermis and should assist in optimising the levels of active agent retained in the epidermis without allowing penetration of the active agent into the patient's system

The challenge in applying a pharmaceutical topically is to achieve percutaneous penetration of the active agent to the site of treatment, in many cases the epidermis. At the same time it is important that the composition have desirable cosmetic characteristics. Application should be easy, smooth and should result in no irritation, discomfort or inconvenience. Desirably the composition should not leave a residue on the surface of the skin, oily or otherwise. Active agents can be applied in various vehicles such as liquid preparations, mousses, gels, ointments, lotions, creams and pastes. Such compositions are often very viscous requiring substantial rubbing to achieve penetration of the active agent to the affected skin layer, an act which often results in discomfort and further irritation. Non viscous creams and lotions require quick and dextrous application as they are inclined to flow off the site of treatment before penetration of the active agent is achieved. As a solution pharmaceuticals can be difficult to apply because they evaporate due to the heat of the skin surface before penetration to the affected site can be achieved. Mousses are well suited to the topical application of pharmaceuticals. Mousse formulations are typically formulated in a single or multiple phase liquid form and housed in a suitable container together with a propellant which facilitates the expulsion of the formulation from the container thus transforming it into a mousse or foam upon application. A mousse or foam formulation has physical characteristics which are dependent, at least in part, upon the choice and relative amounts of components such as solvents, propellants and surfactants which may be present. The combination of such components will determine the stability of the mousse which may retain its foam-like structure upon application or be "slow-breaking" or "quick breaking". This terminology relates to the behaviour of the foam towards shearing action as is sustained when the foam is rubbed into or spread over a surface onto which it has been dispensed. So-called "quick-breaking" mousses are formulated to minimise early evaporation upon application to the skin because of their viscous construction which nevertheless rapidly disintegrates upon spreading by the user. One beneficial characteristic of mousse vehicles is this semi-solid to solid nature of the foam matrix which allows the product to be applied with the hand in any orientation without the risk of run off. Although mousses can be water-based or hydroalcoholic, typically they are formulated with a high alcohol content which, upon application to the skin of a user, quickly evaporates driving the active agent through the upper skin layers to the site of treatment. It is thought that this action is a result of the defatting of the surface layers of the skin by the alcohol content of the mousse. Thus it is expected that an increase in the alcoholic content will have the effect of driving more active agent into the skin because of the increased defatting action of the alcohol present.

EP0331489 is directed to a quick breaking mousse comprising active agents which are useful in the treatment of acne, such as salicylic acid. In addition to the active agent and propellant, the mousse vehicle comprises an aliphatic alcohol in an amount exceeding 40% w/w of the vehicle, water in amounts up to 40% w/w, a fatty alcohol in amounts less than 10% w/w and a surfactant in amounts of up to 15% w/w.

The Australian Patent 619256 to PARKE DAVIS PTY LTD and SOLTEC RESEARCH PTY LTD is directed to a vehicle which is formulated as a quick breaking mousse. In addition to the active agent and propellant, it has a quick breaking mousse vehicle including an aliphatic alcohol in an amount exceeding 40% w/w of the vehicle, water in amounts up to 40% w/w, a fatty alcohol in amounts less than 10% w/w and a surfactant in amounts of up to 15% w/w.

PCT/GB96/00490, a patent application in the name of MEDEVA PLC is also directed to a quick breaking mousse formulation for delivery of corticosteroids. This mousse also includes an aliphatic alcohol in the amount of 40% w/w or more, water in an amount of 10-40% w/w, fatty alcohol in the amount of up to 10% w/w and a surfactant in an amount of up to 15% w/w. A propellant is added compatible with the remainder of the vehicle.

PCT/AU98/00867, a patent application in the name of SOLTEC RESEARCH PTY LTD also describes a mousse vehicle for delivery of anti fungal active agents, particularly ketoconazole. The mousse vehicle of this application may be ethanolic or aqueous. One foamable composition according to this application includes up to 5% w/w long chain alcohols, up to 5% w/w quaternary compound, up to 10% w/w propylene glycol, up to 5% w/w active agent, up to 90% w/w lower alcohol solvent, up to 5% w/w surfactant, 5-95% w/w water and up to 20% propellant.

In relation to mousses it is generally accepted that high levels of alcohol are required to produce a single phase composition. Single phase compositions are desirable to obviate the need to disperse one phase within another prior to application of the mousse. This is conventionally done by shaking the product. In the absence of adequate shaking the active agent can be unevenly or inadequately dispersed through the composition, or can settle in one phase resulting in unsatisfactory application of the active agent to the site requiring treatment. Whilst the mousse formulation is widely accepted as a convenient form of application, high levels of alcohol are, however, commonly associated with skin irritation.

AU-A-21618/88, to RICHARDSON-VICKS, INC describes an anti acne solution which is hydroalcoholic in nature and additionally includes a taurate surfactant. The specification indicates that this formulation is especially effective in achieving penetration of the salicylic acid active agent to the stratum corneum, but does not facilitate penetration of the active through the skin into the general circulation.

In general terms, it is an object of this invention to provide a vehicle for percutaneous delivery of an active agent which is an alternative to those described in the prior art and which provides both high level penetration of the active agent to the site of treatment, and minimal penetration of the active agent past the skin into general circulation. It is a secondary object to provide a pharmaceutical composition suited to the treatment of acne which is cosmetically acceptable as well as being pharmaceutically effective.

Throughout the specification the term "vehicle" means a composition which has only excipients or components required to carry an active agent, but which itself has no pharmaceutical or therapeutic effect. The term "active agent" means a substance having a pharmaceutical, pharmacological or therapeutic effect in the absence of any excipient. A "pharmaceutical composition" is one having at least one active agent in a vehicle formulated to deliver the active agent to the site of treatment. The term "comprises/comprising" when used in this specification is taken to specify the presence of stated features, integers steps or components but does not preclude the presence or addition of one or more other features, integers, steps, components or groups thereof.

### SUMMARY OF THE INVENTION

To this end there is provided a hydroalcoholic vehicle for percutaneous delivery of Salicylic acid to the epidermis, said vehicle comprising lower alcohol, water and wax-surfactant.

Throughout this specification the term "wax-surfactant" means a substance which is a wax also having surfactant properties, a surfactant also having wax properties, a combination of a wax and a surfactant or a substance having both wax and surfactant properties.

The invention is predicated upon the observation that the vehicles of the invention allow the penetration of surprisingly large quantities of Salicylic acid to the epidermis. In trials, skin models treated with vehicles according to the invention show receptor fluid having similar concentrations of active agent as prior art formulations, and also show that the rate of transferral of active agent into the epidermis appears the same as for prior art compositions. However, the higher concentration of Salicylic acid observed in the epidermis suggests that greater quantities of active agent are made available for transferral from the vehicle according to the invention by virtue of its novel formulation. It is postulated that as the volatile component of the vehicle evaporates from the surface of the skin, the Salicylic acid is concentrated into the remaining non-volatile excipients. This increased concentration may lead to an increase in the diffusion rate of the drug into the skin. It is thought that the role of the wax-surfactant is to decrease the tendency of the drug to precipitate out of solution when the evaporation process has gone so far that the concentration of drug exceeds solubility in the remaining phase. Thus a high level of diffusion occurs as a result of the supersaturated state of the formulation remaining on the skin surface. It is also observed that the vehicle according to the invention has surprisingly low alcohol levels when compared to prior art formulations of this type. In particular, in formulations containing such low levels of alcohol it would be expected that the Salicylic acid would prematurely precipitate onto the surface of the skin limiting the quantity available for penetration. In the formulations of the invention it is observed that this premature precipitation does not occur. One advantage of such low levels of alcohol is the decreased level of skin irritation that may result when compared to prior art formulations.

The wax-surfactants utilisable in the vehicles according to the invention may be one or a combination of compounds having characteristics as defined hereinabove in respect of the term "wax-surfactant", and which are selected from the group consisting of anionic surfactants, cationic surfactants and non ionic surfactants. More preferably, the wax surfactant is one, or a combination of compounds selected from the group of surfactants having wax-like properties and including acyl glutamates, phosphoric acids or salts, acyl isethionates, alkyl ether sulfates, alkyldibenzylmethylammonium salts, heterocyclic ammonium salts, tetraalkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin. Other suitable wax-surfactants maybe identified by reference to standard texts such as for example the Surfactant Encyclopaedia (M.M. Rieger, Allured Publishing Corp, 1993).

In one preferred embodiment of the invention, the wax-surfactant may be a cetearyl alcohol/PEG-20 stearate product.

In a preferred embodiment of this aspect of the invention the lower alcohol may be present in amounts of 5-40%w/w and the water is present in amounts 5-95%w/w. The wax-surfactant may be present in amounts of 0.1-10.0%w/w. The lower alcohol is preferably ethanol but may also be isopropanol.

The vehicle is formulated as a mousse and so desirably additionally comprises foaming agent, structuring agents and a propellant. Suitable foaming agents and propellants will be known to those skilled in the art but may include for example hydrocarbons, such as propane, butane and isobutane, and halogenated hydrocarbons, such as dichlorodifluoro methane, dichlorotetrafluoro ethane, and mixtures thereof. Care should be taken to ensure that the propellant is compatible with each of the other components of the formulation. The structuring agent may be selected according to several criteria: it should be soluble within some or all of the formulation components, it should perform the structuring function at a low concentration thereby leaving minimal post application residue on the skin of the user, it should be of acceptable pharmaceutical or cosmetic grade quality. Typically, structuring agents are soluble in organic solvents and have slight solubility in propellants allowing for partial precipitation of solid material hence imparting structure and stability to the foam. Suitable structuring agents include one or more substances which are selected from the group consisting of anionic surfactants, cationic surfactants and non ionic surfactants. More preferably, the structuring agents is one, or a combination of compounds selected from the group of acyl glutamates, phosphoric acids or salts, acyl isethionates, alkyl ether sulfates, alkyldibenzylmethylammonium salts, heterocyclic ammonium salts, tetraalkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin. Other suitable structuring agents may be identified by reference to standard texts such as for example the Surfactant Encyclopaedia (M.M. Rieger, Allured Publishing Corp, 1993). Wax surfactants according to the invention may also be suitable as structuring agents in this embodiment of the invention thereby having a dual role in this invention.

The vehicles may also include other excipients such as for example buffering agents, preservatives, emollients, fragrance, and penetration enhancers.

Described herein is a pharmaceutical composition comprising at least Salicylic acid as active agent in a hydroalcoholic vehicle for percutaneous delivery of the active agent to the epidermis, said vehicle comprising lower alcohol, water and wax-surfactant.

The wax-surfactants utilisable in this aspect of the invention may be one or a combination of compounds having characteristics as defined hereinabove in respect of the term "wax-surfactant", and which are selected from the group consisting of anionic surfactants, cationic surfactants and non ionic surfactants. The wax surfactant may be one, or a combination of compounds selected from the group of surfactants having wax-like properties and including acyl glutamates, phosphoric acids or salts, acyl isethionates, alkyl ether sulfates, alkyldibenzylmethylammonium salts, heterocyclic ammonium salts, tetraalkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin. Other suitable wax-surfactants maybe identified by reference to standard texts such as for example the Surfactant Encyclopaedia (M.M. Rieger, Allured Publishing Corp, 1993).

The wax-surfactant may be a cetearyl alcohol/PEG-20 stearate product.

Pharmaceutical active agents commonly used to treat acne and other skin diseases which may be included in the compositions include the therapeutic substances salicylic acid, isotretinoin, benzoyl peroxide, resorcinol, non-steroidal anti-inflammatory drugs such as ketoprofen, corticosteroids such as cortisone, antifungals, antibiotics for microbial infections and anti-psoriatics such as etrinate. It is common to also include other substances such as anaesthetics such as lignocaine where necessary.

The lower alcohol may be present in amounts of 5-40%w/w and the water may be present in amounts 5-95%w/w. The wax-surfactant may be present in amounts of 0.1-10.0%w/w. The lower alcohol is preferably ethanol but may also be isopropanol, .

The vehicle is formulated as a mousse and so desirably additionally comprises a foaming agent, structuring agents and a propellant. Suitable foaming agents and propellants will be known to those skilled in the art. The structuring agents may be selected according to the criteria mentioned hereinabove. The compositions may also include other excipients such as for example buffering agents, preservatives, emollients, fragrance, and penetration enhancers.

In the invention there is provided a pharmaceutical composition for treatment of acne comprising salicylic acid in a hydroalcoholic vehicle for percutaneous delivery to the epidermis, said vehicle comprising lower alcohol, water and wax-surfactant.

The wax-surfactants utilisable in the vehicles according to the invention may be one or a combination of compounds having characteristics as defined hereinabove in respect of the term "wax-surfactant", and which are selected from the group consisting of anionic surfactants, cationic surfactants and non ionic surfactants. More preferably, the wax surfactant is one, or a combination of compounds selected from the group of surfactants having wax-like properties and including acyl glutamates, phosphoric acids or salts, acyl isethionates, alkyl ether sulfates, alkyldibenzylmethylammonium salts, heterocyclic ammonium salts, tetraalkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin. Other suitable wax-surfactants maybe identified by reference to standard texts such as for example the Surfactant Encyclopaedia (M.M. Rieger, Allured Publishing Corp, 1993).

In one embodiment of the invention the wax-surfactant is a cetearyl alcohol/PEG-20 stearate product.

In a preferred embodiment of the invention the lower alcohol may be present in amounts of 5-40%w/w and the water may be present in amounts of 5-95%w/w. The wax-surfactant may be present in amounts of 0.1-10.0%w/w. The salicylic acid may be present in amounts of 1.0-10.0%w/w.

In a further preferred embodiment of the invention the lower alcohol is preferably ethanol but may also be isopropanol.

The vehicle is formulated as a mousse and so desirably additionally comprises a foaming agent, structuring agents and a propellant. Suitable foaming agents and propellants will be known to those skilled in the art. The structuring agents may be selected according to the criteria mentioned hereinabove. The compositions may also include other excipients such as for example buffering agents, preservatives, emollients, fragrance, and penetration enhancers.

A preferred pH range of the salicylic acid containing pharmaceutical compositions according to the invention is 2.5-6.5.

One particularly preferred embodiment of this aspect of the invention is a salicylic acid mousse composition with the following parameters:

| **Component** | **% w/w** |
|---|---|
| WATER | 0.5 - 95% |
| SODIUM HYDROXIDE | 0 - 3.0% |
| SALICYLIC ACID | 1.0 - 10.0% |
| QUATERNIUM-52 (and) WATER | 0.1 - 5.0% |
| ALCOHOL DENAT. | 5.0 - 40.0% |
| PROPYLENE GLYCOL | 0 - 10.0% |
| CETEARYL ALCOHOL (and) PEG-20 STEARATE | 0.1 - 10.0% |
| FRAGRANCE | 0.05 - 1.0% |
| PROPANE (and) BUTANE (and) ISOBUTANE | 1.0 - 10.0% |

In another aspect of the invention there is provided a pharmaceutical composition comprising salicylic acid in a hydroalcoholic vehicle for percutaneous delivery of an active agent to the epidermis said vehicle comprising lower alcohol, water and wax-surfactant, for use in treating acne by applying to the skin of a patient requiring such treatment an effective amont of said pharmaceutical composition.

The wax-surfactants utilisable in this embodiment of the invention may be one or a combination of compounds having characteristics as defined hereinabove in respect of the term "wax-surfactant", and which are selected from the group consisting of anionic surfactants, cationic surfactants and non ionic surfactants. More preferably, the wax surfactant is one, or a combination of compounds selected from the group of surfactants having wax-like properties and including acyl glutamates, phosphoric acids or salts, acyl isethionates, alkyl ether sulfates, alkyldibenzylmethylammonium salts, heterocyclic ammonium salts, tetraalkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin. Other suitable wax-surfactants maybe identified by reference to standard texts such as for example the Surfactant Encyclopaedia (M.M. Rieger, Allured Publishing Corp, 1993).

In one embodiment of the invention, the wax-surfactant is a cetearyl alcohol/PEG-20 stearate product.

In a preferred embodiment of the invention the lower alcohol may be present In amounts of 5-40%w/w and the water may be present in amounts of 5-95%w/w. The wax-surfactant may be present in amounts of 0.1-10.0%w/w. The salicylic acid may be present in amounts of 1.0-10.0%w/w.

In a further preferred embodiment of the invention the lower alcohol is preferably ethanol but may also be isopropanol.

The pharmaceutical composition is formulated as a mousse and so desirably additionally comprises a foaming agent, structuring agents and a propellant. Suitable foaming agents and propellants will be known to those skilled in the art. The structuring agents may be selected according to the criteria mentioned hereinabove. The compositions may also include other excipients such as for example buffering agents, preservatives, emollients, fragrance, and penetration enhancers.

A preferred pH range of the salicylic acid containing pharmaceutical compositions according to the invention is 2.5-6.5.

Described herein is the use of a vehicle as described above for the delivery of salicylic acid to the skin of a patient for the treatment of acne, said vehicle comprising lower alcohol, water and wax-surfactant. Preferably the wax-surfactant is a cetearyl alcohol/PEG-20 stearate product.

The wax-surfactants may be one or a combination of compounds having characteristics as defined hereinabove in respect of the term "wax-surfactant", and which are selected from the group consisting of anionic surfactants, cationic surfactants and non ionic surfactants. More preferably, the wax surfactant is one, or a combination of compounds selected from the group of surfactants having wax-like properties and including acyl glutamates, phosphoric acids or salts, acyl isethionates, alkyl ether sulfates, alkyldibenzylmethylammonium salts, heterocyclic ammonium salts, tetraalkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin. Other suitable wax-surfactants maybe identified by reference to standard texts such as for example the Surfactant Encyclopaedia (M.M. Rieger, Allured Publishing Corp, 1993).

The wax-surfactant may be a cetearyl alcohol/PEG-20 stearate product.

The lower alcohol may be present in amounts of 5-40%w/w and the water may be present in amounts of 5-95%w/w. The wax-surfactant may be present in amounts of 0.1-10.0%w/w. The salicylic acid may be present in amounts of 1.0-10.0%w/w.

The lower alcohol is preferably ethanol but may also be isopropanol or any other suitable lower alcohol.

The vehicle is formulated as a mousse and so desirably additionally comprises a foaming agent, structuring agents and a propellant. Suitable foaming agents and propellants will be known to those skilled in the art. The structuring agents may be selected according to the criteria mentioned hereinabove. The compositions may also include other excipients such as for example buffering agents, preservatives, emollients, fragrance, and penetration enhancers.

A preferred pH range of the salicylic acid containing pharmaceutical compositions according to the invention is 2.5-6.5.

### EXAMPLE 1

### COMPARATIVE EXAMPLE

In order to determine the superior utility of vehicles and compositions according to the invention, the following salicylic acid mousse formulation according to the invention was prepared:

| **Component** | **% w/w** | **(Trade name)** |
|---|---|---|
| WATER | 58.22 | Purified Water B.P. |
| SODIUM HYDROXIDE | 0.58 | Sodium Hydroxide N.F. |
| SALICYLIC ACID | 2.00 | Salicylic Acid U.S.P. |
| QUATERNIUM-52 (and) WATER | 1.00 | Dehyquart SP |
| ALCOHOL DENAT. | 30.00 | Dehydrated Alcohol U.S.P. |
| PROPYLENE GLYCOL | 2.00 | Propylene Glycol U.S.P. |
| CETEARYL ALCOHOL (and) PEG-20 STEARATE | 1.00 | Polawax GP200 |
| FRAGRANCE | 0.20 | Fragrance A922906 |
| PROPANE (and) BUTANE (and) ISOBUTANE | 5.00 | P45 Hydrocarbon Propellant |

This formulation was manufactured according to the following protocol.

### PRODUCTION OF AEROSOL BASE - ETHANOL PHASE

Check weigh Ethanol, transfer to a suitably sized mixing vessel and heat to 30°C. Add Dehyquart SP, Propylene Glycol and Polawax GP200, maintain at 30°C and stir until clear. Maintain between 25°C-30°C, correct for any loss of Ethanol and add fragrance A922906. Mix until uniform.

### PRODUCTION OF AEROSOL BASE - WATER PHASE

Check weigh Water, transfer to a suitably sized mixing vessel and warm to 50°C. Add Sodium Hydroxide and mix until dissolved. Add Salicylic Acid and mix until dissolved. Cool to between 25°C and 30°C.

### FILLING AND GASSING OF AEROSOL CAN

Filter the Ethanol phase through 100 micron screen. Filter the Water Phase through 100 micron screen. Fill required weight of Ethanol Phase at 25°C-30°C into Can. Fill required weight of Water Phase at 25°C-30°C into Can. Place Valve onto filled Can and crimp. Gas Can with Propellant to required weight.

The aim of the present study was to determine and compare the in-vitro human epidermal penetration and retention of salicylic acid applied topically in two different formulations, one according to the invention and one according to the prior art.

### MATERIALS

### Salicylate Formulations

1. Salicylate mousse formulation as set out hereinabove.
2. Neutrogena ™ Clear Pore treatment formulated as 2% salicylic acid (active ingredient) in a base of purified water, PEG-32, PVM/MA Decadiene crosspolymer, sodium hydroxide and fragrance.

### Human Epidermal Membrane

Epidermal membranes were prepared from full-thickness abdominal skin from 3 female donors (1 (code 136) = 37 years, diffusion cells 1-4 and 13-16; 2 (code 143) = 30 years, diffusion cells 5-8 and 17-20; 3 (code 121) = 56 years, diffusion cells 9-12 and 21-24), obtained following abdominoplasty, using the heat-separation method.

### Other Reagents

All reagents used for the preparation of buffers were of analytical grade and HPLC grade solvents were used throughout for the analysis of salicylic acid.

### TEST PROCEDURES

### Formulation Release Studies

| | |
|---|---|
| Diffusion cells: | Horizontal Franz -type glass cells, application area 1.3cm² |
| Membrane: | Human epidermal membrane |
| Receptor phase: | PBS pH 7.4 + 4% bovine serum albumin @ 35°C (approximately 3.5ml per cell see Table 1) |
| Donor phase: | Finite (approximately 5mg/cm²), unoccluded formulation |
| Duration: | 24 hours with complete receptor phase removal and replacement @ 1, 2, 4, and 24hrs, and 500µl removal and replacement @ 8hr. |
| Mass Balance: | Salicylic acid remaining on the surface of the epidermis, within the first tape strip (designated 'unpenetrated'), within the epidermal membrane and the receptor cell determined at 24hrs. |

At t=0 approx. 5mg/cm² of test formulation (Table 1) was added to the donor side of each cell (n=12 per formulation), using a round ended glass rod was gently wiped over the surface of the membrane to spread formulation as evenly as possible. Concentrations of salicylic acid in each of the samples (receptor phase, remaining on epidermis (washed with 0.5ml 50:50 acetonitrile:distilled water), on first tape strip and within the epidermis) were determined by HPLC.

**Table 1. Cell receptor volumes and Formulation application weights.**

| **Salicylate Mousse** | | | **Neutrogena** | | |
|---|---|---|---|---|---|
| Cell No | Receptor Volume (ml) | Applied per cell | Cell No | Receptor Volume (ml) | Applied per cell |
| 1 | 3.8 | 9µl | 13 | 3.4 | 10µl |
| 2 | 3.6 | " | 14 | 3.6 | " |
| 3 | 3.7 | " | 15 | 3.4 | " |
| 4 | 3.6 | " | 16 | 3.6 | " |
| 5 | 3.6 | " | 17 | 3.7 | " |
| 6 | 3.8 | " | 18 | 3.7 | " |
| 7 | 3.7 | " | 19 | 3.6 | " |
| 8 | 3.6 | " | 20 | 3.7 | " |
| 9 | 3.6 | " | 21 | 3.6 | " |
| 10 | 3.6 | " | 22 | 3.7 | " |
| 11 | 3.6 | " | 23 | 3.6 | " |
| 12 | 3.7 | " | 24 | 3.7 | " |
| Mean±SD g applied/cell | | 0.0077±0. 0005 | Mean±SD g applied/cell | | 0.0078±0.0 003 |

### RESULTS

### Membrane Release of Salicylic Acid

The cumulative amount of salicylic acid entering the receptor phase of each cell, adjusted for the variations in cell receptor volumes, with time is shown in Table 2. The mean data±SEM for each formulation is summarised in figure 1.

**Table 2. Cumulative concentration of salicylate in the receptor phase following 24 hours diffusion from the Salicylate Mousse and Neutrogena Gel.**

| Salicylate Mousse | | | | | |
|---|---|---|---|---|---|
| | Cumulative amount of salicylate (µg) in receptor phase | | | | |
| Cell | 1 hr | 2 hr | 4 hr | 8 hr | 24 hr |
| 1 | 7.11 | 9.16 | 11.01 | 13.15 | 16.04 |
| 2 | 2.83 | 4.58 | 6.33 | 8.42 | 10.39 |
| 3 | 4.87 | 6.136 | 8.46 | 10.48 | 12.62 |
| 4 | 3.00 | 4.74 | 6.49 | 8.24 | 9.64 |
| 5 | 4.55 | 6.70 | 8.72 | 10.75 | 12.71 |
| 6 | 4.0 | 6.14 | 11.01 | 16.10 | 18.13 |
| 7 | 4.49 | 8.17 | 22.14 | 28.03 | 32.67 |
| 8 | 4.43 | 6.27 | 15.96 | 20.85 | 23.25 |
| 9 | 2.30 | 4.04 | 5.79 | 7.67 | 8.70 |
| 10 | 4.57 | 6.31 | 8.10 | 9.85 | 10:53 |
| 11 | 3.35 | 5.10 | 7.03 | 8.85 | 9.44 |
| 12 | 2.80 | 4:60 | 6.70 | 8.91 | 11.64 |
| Mean±SD | 4.02+/-1.30 | 6.04 +/-1.54 | 9.81+/-4.82 | 12.61 +/- 6.20 | 14.65+/- 7.91 |
| | | | | | |

| Neutrogena | | | | | |
|---|---|---|---|---|---|
| | Cumulative amount of salicylate (µg) in receptor phase | | | | |
| Cell | 1 hr | 2 hr | 4 hr | 8 hr | 24 hr |
| 13 | 2.61 | 4.71 | 6.63 | 9.86 | 12.84 |
| 14 | 2.16 | 4.27 | 6.91 | 10.09 | 13.25 |
| 15 | 2.45 | 4.59 | 7.45 | 10.79 | 14.32 |
| 16 | 8.54 | 11.36 | 14.65 | 18.61 | 22.37 |
| 17 | 2.70 | 5.00 | 7.46 | 10.40 | 12.73 |
| 18 | 5.08 | 7.64 | 10.33 | 13.30 | 16.15 |
| 19 | 2.47 | 4.52 | 6.90 | 9.61 | 11.42 |
| 20 | 5.73 | 8.13 | 10:55 | 13.45 | 18.13 |
| 21 | 2.35 | 4.46 | 7.06 | 9.94 | 12.51 |
| 22 | 2.43 | 4.39 | 6.62 | 9.27 | 11.72 |
| 23 | 2.07 | 3.96 | 5.90 | 8.29 | 10.67 |
| 24 | 1.80 | 3.86 | 5.95 | 8.54 | 10.64 |
| Mean±SD | 3.36 +/- 2.04 | 5.57+/- 2.28 | 8.03 +/- 2.57 | 10.85 +/- 2.88 | 13.73 +/- 3.28 |

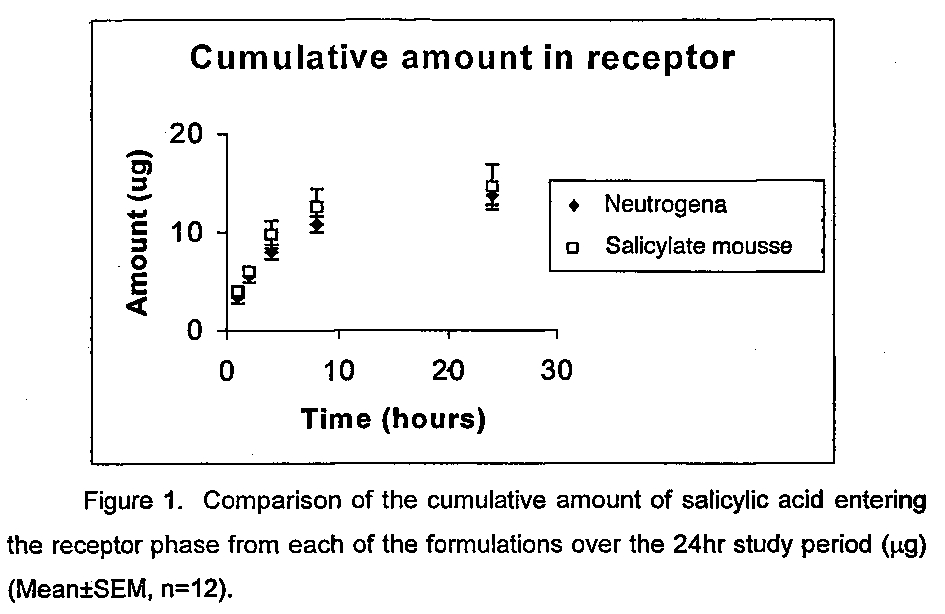

### Mass Balance

The amount of salicylate (µg) and the percent of the applied dose remaining on the surface of the epidermal membrane, recovered from the first tape strip, remaining within the epidermis and receptor phase at 24hrs is shown in Table 3, together with calculation of the total estimated recovery of the applied dose.

**Table 3. Cumulative amount (µg) and percent of applied dose of salicylate recovered at 24 hours from the surface (remaining formulation), tape strip (designated as unabsorbed), epidermis and receptor phase.**

| Salicylate Mousse | | | | | | | | | | |
|---|---|---|---|---|---|---|---|---|---|---|
| Amount of salicylate recovered | | | | | | | | | | |
| Cell | Surface | | Tape-strip | | Epidermis | | Receptor | | Total | |
| | µg | % | µg | % | µg | % | µg | % | µg | % |
| 1 | 93.7 | 58.0 | 1.0 | 0.62 | 15.8 | 9.78 | 16.0 | 9.9 | 126.6 | 78.3 |
| 2 | 99.7 | 61.7 | 1.2 | 0.71 | 7.9 | 4.91 | 10.4 | 6.4 | 119.2 | 73.7 |
| 3 | 122.6 | 75.8 | 1.5 | 0.94 | 7.8 | 4.82 | 12.6 | 7.8 | 144.6 | 89.4 |
| 4 | 99.5 | 61.5 | 2.6 | 1.60 | 4.9 | 3.00 | 9.6 | 6.0 | 116.6 | 72.1 |
| 5 | 127.9 | 79.1 | 1.4 | 0.87 | 7.5 | 4.65 | 12.7 | 7.9 | 149.5 | 92.5 |
| 6 | 74.8 | 46.2 | 3.7 | 2.29 | 10.9 | 6.71 | 18.1 | 11.2 | 107.4 | 66.4 |
| 7 | 72.7 | 45.0 | 5.0 | 3.09 | 10.7 | 6:61 | 32.7 | 20.2 | 121.1 | 74.9 |
| 8 | 74.7 | 46.2 | 5.3 | 3.27 | 10.6 | 6.58 | 23.2 | 14.4 | 113.9 | 70.4 |
| 9 | 95.0 | 58.7 | 4.5 | 2.81 | 11.1 | 6.87 | 8.7 | 5.4 | 119.3 | 73.8 |
| 10 | 96.0 | 59.4 | 5.4 | 3.34 | 11.0 | 6.83 | 10.5 | 6.5 | 123.0 | 78.1 |
| 11 | 112.8 | 69.8 | 3.7 | 2.30 | 8.6 | 5.33 | 9.4 | 5.8 | 134.6 | 83.2 |
| 12 | 102.2 | 63.2 | 4.9 | 3.03 | 9.2 | 5.67 | 11:6 | 7.2 | 127.9 | 79.1 |
| Mean ±SD | 97.6 ± 17.8 | 60.4 ± 11.0 | 3.4 ± 1.7 | 2.1 ± 1.1 | 9.7 ±2.7 | 6.0 ± 1.7 | 14.7 ± 7.1 | 9.1 ± 4.4 | 129.3 ±18.6 | 77.5 ± 7.6 |
| 13 | 158.8 | 98.2 | 1.88 | 1.16 | 3.40 | 2.10 | 12.8 | 7.9 | 176.9 | 109.4 |
| 14 | 144.5 | 89.4 | 3.19 | 1.97 | 2.69 | 1.66 | 13.3 | 8.2 | 163.7 | 101.2 |
| 15 | 152.7 | 94.4 | 4.18 | 2.59 | 3.29 | 2.03 | 14.3 | 8.9 | 174.5 | 107.9 |
| 16 | 169.0 | 104.5 | 1.31 | 0.81 | 2.79 | 1.73 | 22.4 | 13.8 | 195.5 | 120.9 |
| 17 | 151.9 | 93.9 | 1.27 | 0.79 | 3.25 | 2.01 | 12.7 | 7.9 | 169.1 | 104.6 |
| 18 | 170.0 | 105.1 | 1.13 | 0.70 | 2.74 | 1.70 | 16.2 | 10.0 | 190.0 | 117.5 |
| 19 | 140.4 | 86.8 | 2.18 | 1.35 | 3.18 | 1.96 | 11.4 | 7.1 | 157.2 | 97.2 |
| 20 | 163.1 | 100.9 | 0.97 | 0.60 | 2.42 | 1.50 | 16.1 | 10.0 | 182.6 | 112.9 |
| 21 | 191.2 | 118.2 | 1.32 | 0.81 | 3.84 | 2.37 | 12.5 | 7.7 | 208.9 | 129.2 |
| 22 | 152.3 | 94.2 | 1.17 | 0.72 | 3.06 | 1.89 | 11.7 | 7.2 | 168.2 | 104.0 |
| 23 | 215.9 | 133.5 | 1.75 | 1.08 | 4.08 | 2.52 | 10.7 | 6.5 | 232.4 | 143.7 |
| 24 | 152.2 | 94.1 | 1.89 | 1.17 | 2.95 | 1.82 | 10.6 | 6.6 | 167.7 | 103.7 |
| Mean ± SD | 163.5 ±21.3 | 101.1 ±13.2 | 1.9 ± 1.0 | 1.2 ± 0.6 | 3.1 ± 0.5 | 1.9 ± 0.3 | 13.7 ± 3.3 | 8.5 ± 2.0 | 182.2 ± 21.6 | 112.7 ± 13.3 |

### STATISTICAL ANALYSIS

Statistical analysis of the percent of the applied dose of salicylate accumulated in the receptor phase after 24 hours of diffusion indicated there is no statistically significant difference between the two formulations. Statistical analysis of the percent of the applied dose accumulated in the epidermis after 24 hours of diffusion indicated there is a statistically significant difference between the two formulations. The means and probability values are shown in Table 4. Boxplots (Figures 2A and B) indicate that the product according to the invention results in amounts of salicylate in the receptor and epidermis following 24 hours of diffusion which are considerably more variable than those from the Neutrogena product.

**Table 4. Probability values obtained by statistical analysis of the amount of salicylic acid released from the formulations into the receptor and the epidermis at 24 hours.**

| | Product | n | Mean | Std. Deviation | Std. Error Mean | t test | Mann Whitney U |
|---|---|---|---|---|---|---|---|
| % of applied | Salicylate | 12 | 9.06 | 4.38 | 1.27 | t=0.41 | U=58.5 |
| dose assayed | Mousse | | | | | df=22 | |
| in receptor after 24 hours | Neutrogena | 12 | 8.49 | 2.02 | 0.58 | p=0.688 | p=0.435 |
| % of applied | Salicylate | 12 | 5.98 | 1.68 | 0.48 | t=8.22 | U=0 |
| dose assayed | Mousse | | | | | df=11.7* | |
| in epidermis after 24 hrs | Neutrogena | 12 | 1.94 | 0.29 | 0.08 | p<0.001 | p<0.001 |

| | | | | | | | |
|---|---|---|---|---|---|---|---|
| *unequal variances | | | | | | | |

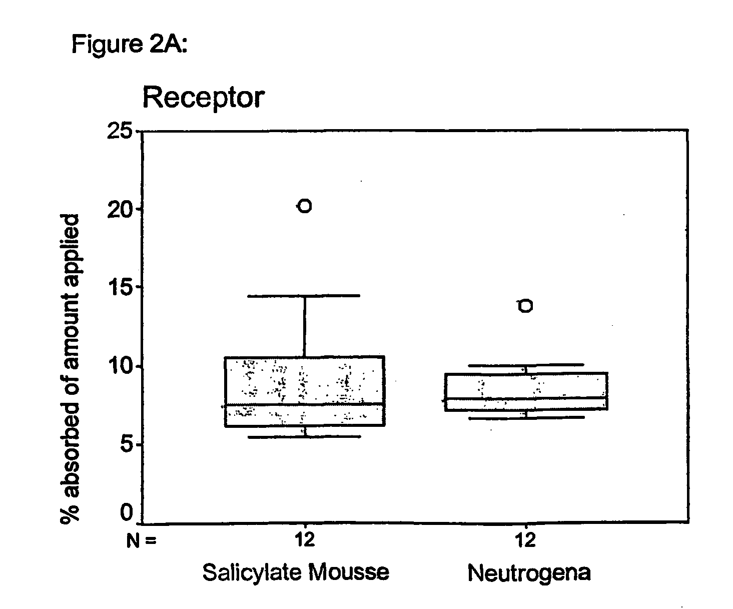

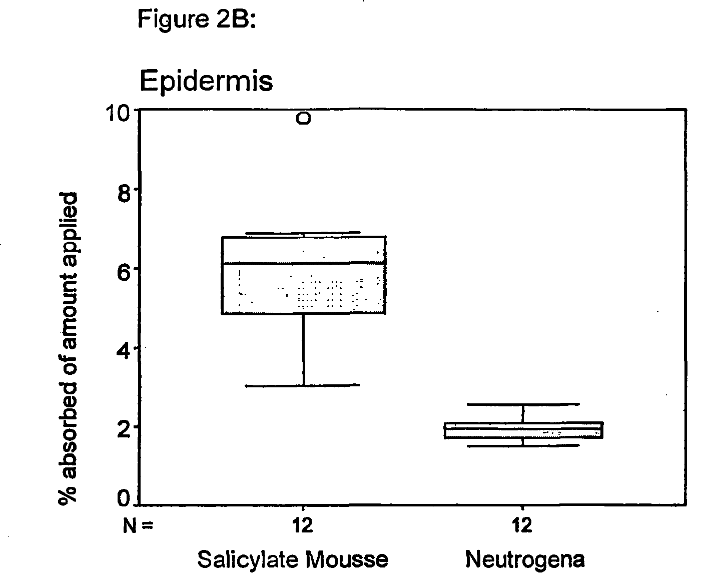

Figures 2A And B Are boxplot representations of the amount of salicylate assayed following 24 Hours of diffusion in the receptor and epidermis respectively. The black line represents the median, the box the interquartile range (50% of the data points), the circles (outliners) are greater than 1.5 but less than 3 times the box length and the whiskers are the range excluding the outliners.

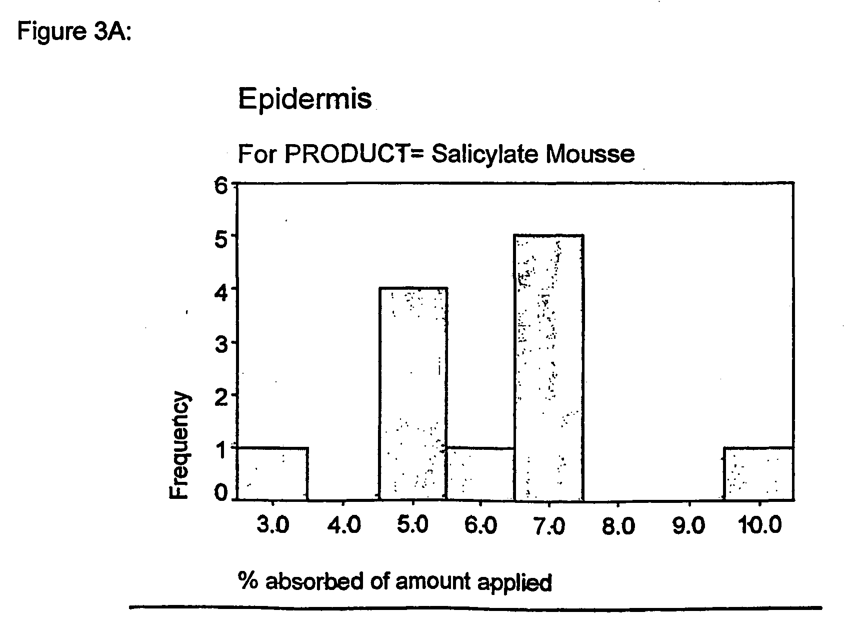

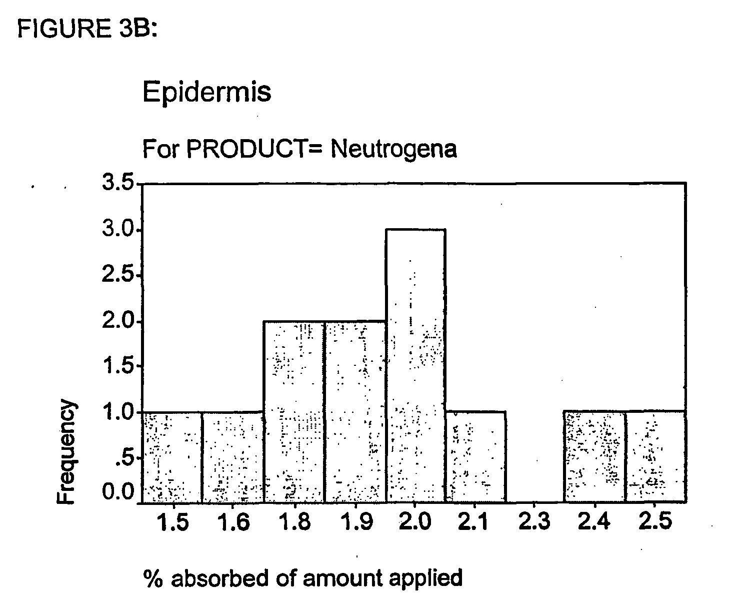

Figures 3A and 3B are histograms representing the distribution of salicylate within the epidermis following diffusion of the salicylate mousse according to the invention and the Neutrogena gel respectively over 24 hours.

### CONCLUSIONS

1. Salicylate from the two formulations accumulated in the receptor phase over 24 hours to the same extent.
2. Salicylate from the mousse accumulated in the epidermis to a greater extent than from the Neutrogena gel.
3. The degree of spread in the data points from the mousse was greater than from the Neutrogena gel.

Examples 2 and 3 demonstrate alternative salicylate formulations according to the invention.

### Example 2

| **Component** | **% w/w** | **(Trade name)** |
|---|---|---|
| SODIUM HYDROXIDE | 0.55 | |
| WATER | 46.55 | |
| PROPYLENE GLYCOL | 2.00 | |
| CETEARYL ALCOHOL (and) PEG-20 STEARATE | 2.50 | Polawax GP200 |
| QUATERNIUM-52 and WATER | 1.00 | Dehyquart SP |
| SALICYLIC ACID | 2.00 | |
| ETHANOL | 40.00 | Alcohol 100 HGF3 |
| PRESERVATIVE | 0.10 | Nipastat |
| PRESERVATIVE | 0.10 | Germall II |
| PERFUME | 0.20 | LFC 38314 |
| PROPANE/BUTANE | 5.00 | P45 |

### Example 3

| **Component** | **% w/w** | **(Trade name)** |
|---|---|---|
| WATER | 59.10 | |
| CETEARYL ALCOHOL and PEG 20 STEARATE | 2.50 | Polawax GP200 |
| QUARTERNIUM-52 and WATER | 1.00 | Dehyquart S.P. |
| SALICYLIC ACID | 2.00 | |
| PRESERVATIVE | 0.10 | Nipastat |
| PRESERVATIVE | 0.10 | Germall II |
| PERFUME | 0.20 | LFC 38314 |
| ETHANOL | 30.00 | Alcohol 95 PGF6 |
| PROPANE/BUTANE | 5.00 | P45 |

### Example 4

The following study was conducted to demonstrate the utility in the preferred mousse formulations according to the invention of different wax-surfactants, and to identify the level at which the wax-surfactant could be incorporated in the preferred mousse formulations of the invention. A secondary benefit in the formulations of the invention wherein the preferred wax-surfactant is cetearyl alcohol/PEG-20 stearate is that it gives structure to the mousse and increases the mousse stability. Alternative wax-surfactants were therefore identified as preferably having, and being present in quantities so as to achieve, this secondary advantage.

### PROCEDURES

### Method of Manufacture

1. Preparation of the bulk ethanol aerosol base
   Weigh Ethanol and transfer into a suitably sized beaker, then add
   Dehyquart and propylene glycol. Stir the solution and heat at 30°C.
   Bulk ethanol base was also made without Dehyquart SP (Quaternium 52.)
2. Preparation of the bulk water aerosol phase
   Weigh water and transfer to a suitably sized beaker
   Add Sodium Hydroxide and mix until dissolved
   Add Salicylic Acid, heat the solution to 50°C and mix until dissolved
3. Preparation of the ethanol phase (Formulations with Dehyquart)
   Weigh the bulk ethanol aerosol base into a beaker, add the require amount of the wax, mix until dissolved at 30°C
4. Filling and Gassing of Aerosol Bottle (Formulations with Dehyquart)
   Transfer ethanol phase to an aerosol bottle
   Transfer water phase to the aerosol bottle
   Seal the can
   Add the Propellant P45
5. Filling and Gassing of Aerosol Bottle (Formulations without Dehyquart)
   Add wax surfactant directly to aerosol bottle
   Transfer ethanol phase (without Dehyquart) to the aerosol bottle
   Transfer water phase to the aerosol bottle
   Seal the can
   Add the Propellant P45

### FORMULATION INGREDIENTS

Two base formulations were used to examine the wax surfactants as shown in the following tables, 5 and 6.

**Table 5: Formulations that contained Dehyquart SP and the wax surfactant**

| **Item No.** | **Ingredient** | **Lot #** | **% w/w** | **Theoretical Mass Weighed (g)** |
|---|---|---|---|---|
| | **Phase 1- Water phase** | | | |
| 1 | Purified water | | 58.42 | 29.21 |
| | | | | |
| 2 | Sodium hydroxide | 13203 | 0.58 | 0.29 |
| 3 | Salicylic acid | 12911 | 2.00 | 1.00 |
| | **Phase 2- Ethanol phase** | | | |
| 4 | Ethanol 100AGF4 | 20102 | 30.00 | 15.00 |
| 5 | Dehyquart SP | 10665 | 1.00 | 0.60 |
| 6 | Propylene Glycol | 98599 | 2.00 | 1.00 |
| 7 | wax | | 1.00 | 0.60 |
| 8 | Fragrance A922906 | 98301 | 0.0 | 0.0 |
| | **Phase 3** | | | |
| 9 | Propellant P45 | | 5 | 2.5 |
| | **TOTAL** | | 100.00 | 50.00 |

**Table 6: Formulations that did not contain Dehyquart SP**

| **Item No.** | **Ingredient** | **Lot #** | **% w/w** | **Theoretical Mass Weighed (g)** |
|---|---|---|---|---|
| | **Phase 1- Water phase** | | | |
| 1 | Purified water | | 58.42 | 29.21 |
| 2 | Sodium hydroxide | 13203 | 0.58 | 0.29 |
| 3 | Salicylic acid | 12911 | 2.00 | 1.00 |
| | **Phase 2- Ethanol phase** | | | |
| 4 | Ethanol 100AGF4 | 20102 | 31.00 | 15.50 |
| 5 | Dehyquart SP | 10665 | 0.00 | 0.00 |
| 6 | Propylene Glycol | 98599 | 2.00 | 1.00 |
| 7 | Wax | | 1.00 | 0.50 |
| 8 | Fragrance A922906 | 98301 | 0.0 | 0.0 |
| | **Phase 3** | | | |
| 9 | Propellant P45 | | 5 | 2.5 |
| | **TOTAL** | | 100.00 | 50.00 |

A listing of the wax surfactants used in the formulation can be found in Table 7.

Formulations containing 0.1%, 0.5%, 1.0%, 5.0%, 7.5% and 10.0% cetearyl alcohol/PEG-20 stearate were made based on the formulation in Table 5. As the level of wax-surfactant was modified from 1.0%, the water level was also modified, while keeping all other ingredients constant.

### Mousse testing procedure

After completing each formulation it was cooled to room temperature. The aerosol bottle was shaken, inverted and the product expelled from the nozzle. The mousse, if formed, was examined for at least 1 minute, and the physical appearance was noted.

The mousse was considered to be stable if the foam structure persisted for at least 1 min.

A description of "good mouse" indicated a full foam with a fine bubble size and creamy to soft texture. Many formulations were initially a "good mouse" from the nozzle but then changed over time.

### Results and discussion

### Formulations with dehyquart

A small group of the wax surfactants were initially examined using the standard formulation that included Dehyquart (see Table 5). The results are listed in Table 7 and indicate that almost all produced an initial good mousse. Some of the foams subsequently broke to a liquid, though most of these were close to a stable mousse. After making a control without a wax surfactant, it was found that the Dehyquart (used as a rust inhibitor) also has good foaming properties, and results in a good mousse that breaks to a liquid in about 20 sec. To examine the foaming and stability properties of the wax surfactants it was necessary to proceed without the interference of the Dehyquart in the formulation.

### Formulations without dehyquart

Dehyquart was removed from the formulation to allow a more thorough examination of the wax surfactants ability to both create and stabilise a foam. All of the wax surfactants were compared in the base formulation described in Table 6. These formulations were compared with a control that contained no wax (134/01/00), a Polawax formulation (134/01/15) and a formulation containing a liquid ethoxylated alcohol(134/02/14). The results are listed Table 7.

The control was expelled as a liquid, which simplified assessing the properties of the other formulations. The liquid surfactant is a good foam former and initially formed a good mousse, but it was not stable and broke to a liquid in 20-25 seconds. The Polawax formulation did not produce as good a mousse as when Dehyquart was also included but the foam was stable.

Therefore, a successful wax surfactant was described as producing a mousse in which a foam structure persisted for at least 1 min. If the formulation did not produce an initial foam, or the foam broke to a liquid in less than 30 sec, it was a clear failure. An intermediate group was also observed and was defined as those where the foam structure lasted for 30-60 sec.

Results are evident from table 7.

**Table 7: Results of Wax surfactant performance in the Salicylic Acid Mousse. √- stable mousse ≈ - intermediate mouse x - no stable mousse**

| **Formulation No** | **CTFA Name** | **Solubility in EtOH** | **Performance of the mousse** | | **Performance of the mousse without Dehyquart in the formulation** | |
|---|---|---|---|---|---|---|
| **134/01/00** | **Control-no wax** | | **Good mousse then quick break to liquid 20 sec** | | **Immediate liquid** | x |
| **134/01/15** | **Cetearyl alcohol/PEG-20 stearate** | **Soluble** | **Good creamy mousse, very** stable | √ | **Flatter mousse with coarser bubbles, small expansion then stable** | √ |
| 134/01/14 | Liquid Surfactant Non-ionic (Ethoxylated nonyl phenol) | Soluble | Good mousse then break to liquid 30-40 sec | ≈ | Good mousse then break to liquid 20-25 sec | **x** |
| 134/02/20 | Sodium hydrogenated tallow glutamate | Soluble | | | Good mousse then very quick collapse to stable thinner foam layer | √ |
| 134/02/01 | Palmitic Acid | Soluble | | | Stable flat foam on a liquid layer | ≈ |
| 134/02/15 | DEA Oleth-3 Phosphate | Soluble | | | Good mousse then slow expansion to coarse bubble then liquid 1 min | √ |
| 134/02/16 | Lecithin | Not soluble | | | Wet coarse mousse, very stable | √ |
| 134/02/07 | Cetearyl Achohol, (and) Dicetyl Phosphate (and) Ceteth-10 Phosphate | Soluble | Stable foam | √ | Out wet then stable coarse Out wet then stable foam forms, coarse bubbles | √ |
| 134/02/11 | Sodium Laureth Sulfate | Partially soluble | | | Good mousse then break to liquid 30 sec | ≈ |
| 134/01/02 | Ammonium lauryl sulfate | Soluble | Good mousse then very slow collapse | √ | Very quick break to liquid | x |
| 134/02/23 | Stearalkonium Chloride | Soluble | | | Good mousse then slow expansion to coarse bubble then liquid 50-60 sec | ≈ |
| 134/02/08 | Cetearyl Achohol and Behentrimonium Methosulphate | Soluble | Stable foam | √ | Out wet then stable foam forms, coarse bubbles | √ |
| 134/02/24 | Cetearyl Alcohol | Soluble | | | Very good creamy mousse, very stabile | √ |
| 134/01/05 | Stearyl Alcohol | Soluble | Good mousse then slow expansion to coarser bubble | √ | Partial mousse, very quick break to thin white layer on liquid | x |
| 134/02/03 | PEG-40 Stearate | Soluble | Stable foam | √ | Good mousse then slow expansion to coarse bubble then liquid 1-2 min | √ |
| 134/01/07 | PEG 20 Glyceryl Stearate | Soluble | Good mousse, slow expansion and wetter | √ | Good foam then slow expansion to coarser bubble, stable | √ |
| 134/02/09 | Glycol Stearate | Soluble | Fine mousse, collapsing in less than 30s | x | Flat coarse mouse, then slowly to large bubble then stable | √ |
| 134/01/08 | Glyceryl Stearate | Partially soluble | Sticky mousse, stable | √ | Precipitate interfered with nozzte, but partial stable mousse formed but | √ |
| 134/01/09 | Polyglyceryl-3-Stearate | Partially soluble | Good mousse, quick break to large bubble then stable | √ | Very quickly expands to a large bubble, then stable | √ |
| 134/01/10 | Sucrose Stearate | Not soluble | Good mousse then rapid collapse to thinner layer stable foam | √ | Good mousse then slight collapse to stable foam | √ |
| 134/02/06 | Polysorbate 61 | Soluble | - | | Out wet then foam forms, slow expansion to liquid 2 min | √ |
| 134/02/10 | Sorbitan Stearate | Soluble | | | Quick expansion to small layer large bubbles on liquid | ≈ |
| 134/02/05 | Ceteareth-20 | Soluble | Stable foam | √ | Good mousse then very slow expansion to coarse bubble then liquid 5-10 min | √ |
| 134/02/02 | PEG-Lanolin | Soluble | | | Good mousse then very slow expansion to coarse bubble then liquid 5-10 min | √ |

It will be appreciated that the vehicles and compositions of the invention as described provide advantages over the prior art both in terms of aesthetic characteristics and medical characteristics by virtue of the high levels of penetration achieved by the active agents.

## Claims

1. A pharmaceutical mousse composition suitable for treatment of acne comprising salicylic acid in a hydroalcoholic vehicle for percutaneous delivery to the epidermis, wherein the salicylic acid is present in an amount of 1.0 - 10 %w/w and wherein said vehicle comprises ethanol or isopropanol in an amount of 5 - 40 %w/w, water, a wax-surfactant and a propellant.

2. The pharmaceutical mousse composition as claimed in claim 1, said wax-surfactant being one, or a combination of compounds selected from the group consisting of acyl glutamates, phosphoric acids or salts, acyl isothionates, alkyl ether sulfates, alkyldibenzylmethylammonium salts, heterocyclic ammonium salts, tetraalkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin.

3. The pharmaceutical mousse composition as claimed in claim 1, wherein said wax-surfactant is cetearyl alcohol/PEG-20 stearate.

4. The pharmaceutical mousse composition as claimed in any one of claims 1 to 3, wherein water is present in an amount of 5-95 %w/w.

5. The pharmaceutical mousse composition as claimed in any one of claims 1 to 4, wherein the wax-surfactant is present in an amount of 0.1 to 10% w/w.

6. The pharmaceutical mousse composition as claimed in any one of claims 1 to 5, wherein the pH range of the composition is 2.5 - 6.5.

7. The pharmaceutical mousse composition as claimed in any one of claims 1 to 6, wherein the propellant is a hydrocarbon propellant.

8. A pharmaceutical mousse composition comprising salicylic acid in a hydroalcoholic vehicle for use in treating acne by applying to the skin, wherein the salicylic acid is present in an amount of 1.0 - 10 %w/w and wherein said vehicle comprises ethanol or isopropanol in an amount of 5 - 40 %w/w, water, a wax-surfactant and a propellant.

9. A pharmaceutical mousse composition for use as claimed in claim 8, said wax-surfactant being one, or a combination of compounds selected from the group consisting of acyl glutamates, phosphoric acids or salts, acyl isothionates, alkyl ether sulfates, alkyldibenzylmethylammonium salts, heterocyclic ammonium salts, tetra-alkylammonium salts, ethoxylated carboxylic acids, ethoxylated glycerides, glycol esters, monoglycerides, polyglyceryl esters, polyhydric alcohol esters and ethers, sorbitan/sorbitol esters and ethoxylated alcohols including lanolin.

10. A pharmaceutical mousse composition for use as claimed in claim 8, wherein said wax-surfactant is cetearyl alcohol/PEG-20 stearate.

11. A pharmaceutical mousse composition for use as claimed in any one of claims 8 to 10, wherein water is present in an amount of 5-95 %w/w.

12. A pharmaceutical mousse composition for use as claimed in any one of claims 8 to 11, wherein the wax-surfactant is present in an amount of 0.1 to 10% w/w.

13. A pharmaceutical mousse composition for use as claimed in any one of claims 8 to 12, wherein the pH range of the composition is 2.5 - 6.5.

14. A pharmaceutical mousse composition for use as claimed in any one of claims 8 to 13, wherein the propellant is a hydrocarbon propellant.

## Patentansprüche

1. Eine pharmazeutische Schaumzusammensetzung (mousse compostion), welche zur Behandlung von Akne geeignet ist, umfassend Salicylsäure in einem wässrigen-alkoholischen Vehikel zur perkutanen Abgabe an die Epidermis, wöbei die Salicylsäure in einer Menge von 1,0 - 10 % Gew./Gew. vorhanden ist und wobei das Vehikel Ethanol oder Isopropanol in einer Menge von 5-40 % Gew./Gew., Wasser, ein oberflächenaktives Mittel aus Wachs und ein Treibmittel umfasst.

2. Die pharmazeutische Schaumzummensetzung wie in Anspruch 1 beansprucht, wobei das oberflächenaktive Mittel aus Wachs eine Verbindung oder eine Kombination von Verbindungen ist, ausgewählt aus der Gruppe bestehend aus Acylglutamaten, Phosphorsäuren oder Salzen, Acylisothionaten, Alkylethersulfaten, Alkyldibenzylmethylammoniumsalzen, heterocyclischen Ammoniumsalzen, Tetraalkylammoniumsalzen, ethoxylierten Carbonsäuren, ethoxylierten Glyceriden, Glycolestern, Monoglyceriden, Polyglycerylestern, Estern und Ethern mehnwertiger Alkohole, Sorbitan-/Sorbitolestem und ethoxylierten Alkoholen, einschließlich Lanolin.

3. Die pharmazeutische Schaumzummensetzung wie in Anspruch 1 beansprucht, wobei das oberflächenaktive Mittel aus Wachs Cetearylalkohol/PEG-20 Stearat ist.

4. Die pharmazeutische Schaumzusammensetzung wie in einem der Ansprüche 1 bis 3 beansprucht, wobei Wasser in einer Menge von 5-95 % Gew./Gew. vorliegt.

5. Die pharmazeutische Schaumzusammensetzung wie in einem der Ansprüche 1 bis 4 beansprucht, wobei das oberflächenaktive Mittel aus Wachs in einer Menge von 0,1 bis 10 % Gew./Gew. vorliegt.

6. Die pharmazeutiche Schaumzusammensetzung wie in einem der Ansprüche 1 bis 5 beansprucht, wobei der pH-Bereich der Zusammensetzung 2,5-6,5 beträgt.

7. Die pharmazeutische Schaumzusammensetzung wie in einem der Ansprüche 1 bis 6 beansprucht, wobei das Treibmittel ein Kohlenwasserstofftreibmittel ist.

8. Eine pharmazeutische Schaumzusammensetzung, umfassend Salicylsäure in einem wässrigen-alkoholischen Vehikel zur Verwendung bei der Behandlung von Akne durch Auftragen auf die Haut, wobei die Salicylsäure in einer Menge von 1,0-10 % Gew./Gew. vorliegt und wobei das Vehikel Ethanol oder Isopropanol in einer Menge von 5-40 % Gew./Gew., Wasser, ein oberflächenaktives Mittel aus Wachs und ein Treibmittel umfasst.

9. Eine pharmazeutische Schaumzusammensetzung zur Verwendung wie in Anspruch 8 beansprucht, wobei das oberflächenaktive Mittel aus Wachs eine Verbindung oder eine Kombination von Verbindungen ist, ausgewählt aus der Gruppe bestehend aus Acylglutamaten, Phosphorsäuren oder Salzen, Acylisothionaten, Alkylethersulfaten, Alkyldibenzylmethylammonuimsalzen, heterocyclischen Ammoniumsalzen, Tetraalkylammoniumsalzen, ethoxylierten Carbonsäuren, ethoxylierten Glyceriden, Glycolestern, Monoglyceriden, Polyglycerylestern, Estern und Ethern mehrwertiger Alkohole, Sorbitan-/Sorbitolestern und ethoxylierten Alkoholen, einschließlich Lanolin.

10. Eine pharmazeutische Schaumzusammensetzung zur Verwendung wie in Anspruch 8 beansprucht, wobei das oberflächenaktive Mittel aus Wachs Cetearylalkohol/PEG-20 Stearat ist.

11. Eie pharmazeutische Schaumzusammensetzung zur Verwendung wie in einem der Ansprüche 8 bis 10 beansprucht, wobei Wasser in einer Menge von 5-95 % Gew./Gew. vorliegt.

12. Eine pharmazeutische Schaumzusammensetzung zur Verwendung wie in einem der Ansprüche 8 bis 11 beansprucht, wobei das oberflächenaktive Mittel aus Wachs in einer Menge von 0,1 bis 10 % Gew./Gew. vorliegt.

13. Eine pharmazeutische Schaumzusammensetzung zur Verwendung wie in einem der Ansprüche 8 bis 12 beansprucht, wobei der pH-Bereich der Zusammensetzung 2,5-6,5 beträgt.

14. Eine pharmazeutische Schaumzusammensetzung zur Verwendung wie in einem der Ansprüche 8 bis 13 beansprucht, wobei das Treibmittel ein Kohlenwasserstofftreibmittel ist.

## Revendications

1. Composition pharmaceutique en mousse apte au traitement de l'acné, comprenant de l'acide salicylique dans un véhicule hydroalcoolique pour administration percutanée à l'épiderme, dans laquelle l'acide salicylique est présent en une quantité de 1,0 à 10 % en poids/poids et dans laquelle ledit véhicule comprend de l'éthanol ou de l'isopropanol en une quantité de 5 à 40 % en poids/poids, de l'eau, un agent tensioactif du type cire et un agent propulseur.

2. Composition pharmaceutique en mousse suivant la revendication 1, ledit agent tensioactif du type cire étant un composé, ou une association de composés choisi(s) dans le groupe consistant en des acylglutamates, des acides phosphoriques ou sels, des acylisothionates, des alkyléthersulfates, des sels d'alkyldibenzylméthylammonium, des sels d'ammonium hétérocycliques, des sels de tétraalkylammonium, des acides carboxyliques éthoxylés, des glycérides éthoxylés, des esters de glycols, des monoglycérides, des esters de polyglycéryle, des esters et éthers d'alcools polyhydroxyliques, des esters de sorbitanne/sorbitol et des alcools éthoxylés, y compris la lanoline.

3. Composition pharmaceutique en mousse suivant la revendication 1, dans laquelle ledit agent tensioactif du type cire consiste en alcool cétéarylique/stéarate de PEG-20.

4. Composition pharmaceutique en mousse suivant l'une quelconque des revendications 1 à 3, dans laquelle l'eau est présente en une quantité de 5 à 95 % en poids/poids.

5. Composition pharmaceutique en mousse suivant l'une quelconque des revendications 1 à 4, dans laquelle l'agent tensioactif du type cire est présent en une quantité de 0,1 à 10 % en poids/poids.

6. Composition pharmaceutique en mousse suivant l'une quelconque des revendications 1 à 5, dans laquelle la plage de pH de la composition va de 2,5 à 6,5.

7. Composition pharmaceutique en mousse suivant l'une quelconque des revendications 1 à 6, dans laquelle l'agent propulseur est un agent propulseur hydrocarboné.

8. Composition pharmaceutique en mousse comprenant de l'acide salicylique dans un véhicule hydroalcoolique, pour son utilisation dans le traitement de l'acné par application à la peau, dans laquelle l'acide salicylique est présent en une quantité de 1,0 à 10 % en poids/poids et dans laquelle ledit véhicule comprend de l'éthanol ou de l'isopropanol en une quantité de 5 à 40 % en poids/poids, de l'eau, un agent tensioactif du type cire et un agent propulseur.

9. Composition pharmaceutique en mousse pour son utilisation suivant la revendication 8, l'agent tensioactif du type cire étant un composé, ou une association de composés choisi(s) dans le groupe consistant en des acylglutamates, des acides phosphoriques ou sels, des acylisothionates, des alkyléthersulfates, des sels d'alkyldibenzylméthylammonium, des sels d'ammonium hétérocycliques, des sels de tétraalkylammonium, des acides carboxyliques éthoxylés, des glycérides éthoxylés, des esters de glycols, des monoglycérides, des esters de polyglycéryle, des esters et éthers d'alcools polyhydroxyliques, des esters de sorbitanne/sorbitol et des alcools éthoxylés, y compris la lanoline.

10. Composition pharmaceutique en mousse pour son utilisation suivant la revendication 8, dans laquelle ledit agent tensioactif du type cire consiste en alcool cétéarylique/stéarate de PEG-20.

11. Composition pharmaceutique en mousse pour son utilisation suivant l'une quelconque des revendications 8 à 10, dans laquelle l'eau est présente en une quantité de 5 à 95 % en poids/poids.

12. Composition pharmaceutique en mousse pour son utilisation suivant l'une quelconque des revendications 8 à 11, dans laquelle l'agent tensioactif du type cire est présent en une quantité de 0,1 à 10 % en poids/poids.

13. Composition pharmaceutique en mousse pour son utilisation suivant l'une quelconque des revendications 8 à 12, dans laquelle la plage de pH de la composition va de 2,5 à 6,5.

14. Composition pharmaceutique en mousse pour son utilisation suivant l'une quelconque des revendications 8 à 13, dans laquelle l'agent propulseur est un agent propulseur hydrocarboné.
